(19) Europäisches Patentamt / European Patent Office / Office européen des brevets

(11) **EP 1 550 480 B1**

(12) **FASCICULE DE BREVET EUROPEEN**

(45) Date de publication et mention
de la délivrance du brevet:
**29.08.2007  Bulletin 2007/35**

(51) Int Cl.:
*A61N 1/368* *(2006.01)*

(21) Numéro de dépôt: **04293141.0**

(22) Date de dépôt: **28.12.2004**

(54) **Dispositif médical implantable actif, notamment stimulateur cardiaque, à gestion perfectionnée des commutations automatiques de mode AAI/DDD en présence de BAV paroxystiques**

Implantierbare medizinische Vorrichtung, wie Schrittmacher, mit verbesserter Steuerung der automatischen AAI/DDD Modusschaltung in Anwesenheit anfallartig auftretender atrio-ventrikulärer Blöcke

Implantable medical device such as a pacemaker, with improved control of automatic AAI/DDD mode switching in the presence of paroxysmal AV block

(84) Etats contractants désignés:
**AT BE BG CH CY CZ DE DK EE ES FI FR GB GR HU IE IS IT LI LT LU MC NL PL PT RO SE SI SK TR**

(30) Priorité: **29.12.2003  FR 0315517**

(43) Date de publication de la demande:
**06.07.2005  Bulletin 2005/27**

(73) Titulaire: **ELA MEDICAL**
**92541 Montrouge (FR)**

(72) Inventeurs:
• **Henry, Christine**
**75014 Paris (FR)**
• **Amblard, Amel**
**92290 Chatenay Malabry (FR)**
• **Limousin, Marcel**
**75014 Paris (FR)**

(74) Mandataire: **Dupuis-Latour, Dominique et al**
**SEP Bardehle Pagenberg Dost Altenburg Geissler**
**14 boulevard Malesherbes**
**75008 Paris (FR)**

(56) Documents cités:
**EP-A- 0 488 904      EP-A- 1 346 750**
**WO-A-99/10044       US-A- 5 893 882**
**US-A1- 2003 078 627  US-B1- 6 343 231**

## Description

L'invention concerne les "dispositifs médicaux implantables actifs" tels que définis par la directive 90/385/CEE du 20 juin 1990 du Conseil des communautés européennes, plus précisément les dispositifs stimulateurs cardiaques, dispositifs "multisite" (triple ou quadruple chambre), défibrillateurs et/ou cardioverteurs, permettant de délivrer au coeur des impulsions électriques de faible énergie pour le traitement des troubles du rythme cardiaque.

Elle concerne plus particulièrement ceux de ces dispositifs qui comprennent des circuits de stimulation et de détection à la fois sur l'oreillette et sur le ventricule et peuvent opérer selon deux modes de fonctionnement, DDD ou AAI. Ces dispositifs peuvent être pourvus d'un mode dénommé "AAISafeR" assurant une commutation automatique du mode DDD au mode AAI et inversement.

Le mode de fonctionnement de base d'un stimulateur DDD/AAI est un mode AAI, avec une stimulation auriculaire simple chambre et une surveillance (détection) de l'activité ventriculaire. Ce mode est maintenu tant que la conduction atrio-ventriculaire est normale, c'est-à-dire tant que chaque événement auriculaire (détection auriculaire, correspondant à une activité spontanée, ou stimulation auriculaire) est suivi d'une détection ventriculaire associée.

Dans certaines circonstances peuvent cependant apparaître des blocs auriculo-ventriculaires (BAV) entraînant un trouble temporaire de dépolarisation du ventricule. Dans ce cas, le stimulateur bascule automatiquement en mode DDD automatique, avec des paramètres optimisés pour cette situation de BAV temporaire. Après disparition du BAV, et donc rétablissement de la conduction auriculo-ventriculaire, dès lors qu'un certain nombre de conditions sont remplies le stimulateur retourne automatiquement au mode AAI.

Cette commutation entre modes DDD et AAI est notamment décrite dans les EP-A-0 488 904 et EP-A-1 346 750 (ELA Médical), ainsi que dans les US-A-2002/0082646 et US-A-2003/0078627 (Medtronic, Inc.).

Le point de départ de l'invention réside dans un certain nombre d'observations effectuées lors d'un suivi clinique de patients appareillés avec des dispositifs DDD/AAI à commutation automatique de mode.

Il est apparu en effet que ces appareils présentent une spécificité insuffisante en fonction du type de BAV, de sorte que dans certains cas se produisent des commutations inappropriées vers le mode DDD, conduisant à des alternances de commutations AAI→DDD puis DDD→AAI non justifiées, ou encore à des commutations définitives, inutiles, en mode DDD.

Plus précisément, on distingue classiquement trois degrés de BAV, correspondant à une gravité croissante du trouble de conduction :

a) le BAV du premier degré (ci-après "BAV1") correspond à une conduction présente, mais retardée ; la commutation vers le mode DDD est déclenchée lorsque le nombre d'évènements auriculaires suivis d'une détection ventriculaire survenant après un délai supérieur à, par exemple, 350 ms (pour un événement auriculaire spontané) ou 450 ms (pour un événement auriculaire stimulé) dépasse un nombre donné, par exemple en cas de détection de six cycles cardiaques consécutifs répondant à ce critère.

b) le BAV du deuxième degré (ci-après "BAV2"), se caractérise par une conduction incomplète, l'allongement progressif de l'intervalle PR (ou AR) étant tel qu'une partie des ondes P ne sont plus conduites. La commutation en mode DDD sur un BAV du deuxième degré est typiquement déclenchée lorsque le nombre d'évènements auriculaires non suivis d'une détection ventriculaire excède un certain nombre sur la durée d'une fenêtre de surveillance s'étendant sur un nombre prédéterminé d'évènements auriculaires : en d'autres termes, la commutation en DDD est par exemple déclenchée lorsque le dispositif détecte trois ondes P bloquées non consécutives parmi les douze derniers cycles cardiaques.

c) le BAV complet, ou BAV du troisième degré (ci-après "BAV3"), le plus grave, se manifeste par des ondes auriculaires (stimulées ou spontanées) totalement bloquées, c'est-à-dire qui ne sont plus suivies de dépolarisations ventriculaires ; le dispositif doit alors opérer rapidement la commutation en mode DDD, cette commutation intervenant typiquement lorsqu'il détecte une succession de deux ondes auriculaires bloquées (détectées ou stimulées), ou s'il s'écoule plus de trois secondes sans détection ventriculaire. Il y a également lieu de prendre en compte :

d) la pause ventriculaire, qu'elle trouve son origine ou non dans un trouble de la conduction auriculo-ventriculaire ; il y a pause ventriculaire lorsque l'intervalle séparant deux événements ventriculaires excède un délai donné, par exemple excède trois secondes.

Après une commutation en mode DDD sur BAV, pour retourner au mode AAI le dispositif attend que soient remplis un certain nombre de critères de retour, par exemple il revient au mode AAI après cent cycles avec stimulation ventriculaire (afin de pouvoir tester, en revenant au mode AAI, si une conduction atrioventriculaire spontanée s'est rétablie) ou douze cycles consécutifs avec détection d'une dépolarisation spontanée du ventricule.

Par ailleurs, il apparaît souhaitable de prévoir une limitation du nombre de commutations successives de AAI vers DDD sur une période donnée.

Si, par exemple, le dispositif a déclenché plus de quinze commutations sur une période de 24 heures ou si, sur une période de trois jours consécutifs, le dispositif a déclenché plus de cinq commutations par 24 heures, alors il bascule définitivement en mode DDD et fonctionne sur la base de paramètres de stimulation préprogrammés (notamment le délai auriculo-ventriculaire), cette configuration étant maintenue jusqu'à nouvel examen du patient et reprogrammation éventuelle par le praticien.

Ces règles de commutation se sont cependant révélées, dans certains cas, inappropriées.

Le premier cas est celui des "troubles du rythme auriculaire" (TdRA), terme générique qui recouvre diverses arythmies auriculaires (épisodes d'accélération non physiologiques du rythme) telles que tachycardie, fibrillation, flutter, etc., troubles tous caractérisés, à la détection par un rythme auriculaire rapide.

Dans un tel cas de TdRA suspecté ou avéré, les critères ci-dessus de détection de BAV1, BAV2 ou BAV3 ne sont plus appropriés, car un même cycle cardiaque laisse généralement apparaître plusieurs événements auriculaires, et les détections P multiples faussent l'analyse de la conduction auriculo-ventriculaire (en revanche, le critère de détection de pause ventriculaire reste pertinent).

Par ailleurs, les BAV présentent très souvent un caractère intermittent, tout particulièrement les BAV1 et BAV2 qui peuvent, dans certaines situations, présenter un caractère simplement paroxystique, c'est-à-dire qu'ils peuvent survenir communément lors, notamment, de phases d'effort ou de sommeil, et disparaître spontanément en fin d'effort ou à l'éveil.

Dans ces situations de BAV paroxystiques, avec les dispositifs connus fonctionnant de la manière que l'on vient d'indiquer on peut constater un certain nombre d'inconvénients :

- une commutation définitive en mode DDD sur détection de BAV paroxystique d'effort ou nocturne n'est pas a *priori* pertinente, car les patients peuvent présenter par ailleurs une conduction atrioventriculaire satisfaisante pendant la journée ou au repos.
- pendant une même phase d'effort, il serait souhaitable d'éviter des successions de commutations AAI→DDD puis DDD→AAI : en effet, s'il y a eu, par exemple, une commutation AAI→DDD à l'effort, on peut raisonnablement penser que le BAV qui a déclenché cette commutation va persister jusqu'à la fin de l'effort, et que les tentatives de recommutation en AAI ne sont pas appropriées tant que l'effort persiste, car elles risquent d'entraîner des pauses symptomatiques lors de cet exercice.
- en revanche, l'effort terminé, il est souhaitable de pouvoir au plus vite retrouver un fonctionnement AAI puisque, si le BAV était seulement paroxystique, la conduction devrait normalement réapparaître.
- il peut être excessif de générer une commutation définitive après des commutations successives très proches les unes des autres (en cas d'épisode de trouble de conduction), car cet épisode peut être unique sur une période donnée, par exemple un épisode de bloc d'une durée d'une heure sur une journée par exemple.
- d'autre part, peu d'informations cliniques étant disponibles sur le caractère "paroxystique" des BAV (leur durée, notamment est mal connue), il est peut-être excessif d'opérer une commutation définitive provoquée par une succession de commutations rapprochées entraînées par un même épisode de conduction. Mais il est pour autant nécessaire de bien discriminer les BAV chroniques, afin de pouvoir être sûr que l'appareil finira par commuter en mode DDD si l'épisode de trouble de conduction dure plusieurs jours.
- enfin, à l'effort le rythme cardiaque s'accélère, et sur un plan hémodynamique il n'est pas souhaitable de laisser s'allonger l'intervalle PR (ou AR) jusqu'à 350 ms (ou 450 ms) avant de déclencher la commutation vers le mode DDD. Ces valeurs de 350 ou 450 ms sont, certes, paramétrables, mais elles sont fixes quelle que soit la fréquence et ne tiennent pas compte, pour la détection d'un BAV1, de l'état particulier du patient, en phase d'effort ou non.

L'invention propose de remédier à ces difficultés en apportant un perfectionnement aux dispositifs connus par un aménagement approprié de la commutation de mode.

Plus précisément, l'un des buts de l'invention vise à introduire une sélectivité dans le fonctionnement afin, d'une part, de gérer de façon particulière le cas des BAV paroxystiques et, d'autre part, de pouvoir assurer une gestion fine tenant compte à la fois du type de BAV et des circonstances ayant entouré la survenue de ce BAV.

Un autre but de l'invention est d'éviter des commutations définitives inappropriées en cas de détection de BAV à l'effort ou en sommeil.

Un autre but encore de l'invention est d'éviter des commutations successives inappropriées susceptibles d'entraîner inutilement une commutation définitive en mode DDD.

Un autre but de l'invention est de gérer de manière plus fine la commutation définitive en mode DDD en opérant au préalable une commutation durable, mais non définitive, en mode DDD, la commutation ne devenant définitive que si des critères particuliers sont vérifiés.

Un autre but de l'invention, enfin, est de gérer le retour vers le mode AAI d'une manière plus efficace sur le plan hémodynamique dans le cas où le patient se trouve dans un état temporaire particulier tel qu'effort ou sommeil, ou encore dans d'autres états diagnostiqués tels qu'une phase de décompensation cardiaque ou une phase d'arythmie.

Le type de dispositif auquel s'applique l'invention est un dispositif DDD-CAM d'un type connu, par exemple selon le US 2003/078627 précité. Les moyens particuliers mis en oeuvre par la présente invention sont explicités dans la partie caractérisante de la revendication 1.

Les sous-revendications visent des modes de réalisation subsidiaires avantageux, concernant notamment :

la prise en compte d'états autres que le sommeil et l'effort;

la présence de compteurs permettant d'assurer une gestion différenciée des basculements de mode ;

la commutation inverse de DDD vers AAI ;

la possibilité d'opérer une commutation durable en mode DDD ;

la possibilité de commander une commutation non plus durable, mais définitive, vers le mode DDD ;

la gestion particulière des commutations à l'effort; et

la possibilité d'enregistrer des données spécifiques permettant de documenter la conduction auriculo-ventriculaire pour diagnostic ultérieur.

On va maintenant décrire un exemple de réalisation du dispositif de l'invention, qui peut être mis en oeuvre par une programmation appropriée du logiciel de commande d'un stimulateur connu de type double chambre intégrant un mode DDD et un mode AAI avec surveillance de l'activité ventriculaire.

L'invention peut notamment être appliquée aux dispositifs commercialisés par ELA Médical, Montrouge, France. Il s'agit de dispositifs à microprocesseur comportant des circuits pour recevoir, mettre en forme et traiter des signaux électriques recueillis par des électrodes implantées. Ils est possible d'y transmettre par télémétrie des logiciels qui seront conservés en mémoire et exécutés pour mettre en oeuvre les fonctions de l'invention qui seront décrites ci-dessous.

*Définitions et fonctionnement selon l'état de la technique*

On va tout d'abord donner un certain nombre de définitions utilisées dans la présente description.

*Détection P :* recueil d'une activité spontanée (onde P) ayant son origine dans l'oreillette ; on considérera qu'il y a effectivement détection P si celle-ci n'est pas suivie dans un délai donné, par exemple dans les 31 ms, par une détection ventriculaire (sinon, on se trouverait dans une situation de *"far-field* ventriculaire", c'est-à-dire de recueil via l'oreillette d'une dépolarisation lointaine provenant du ventricule).

*Détection R :* recueil d'une activité spontanée (onde R) ayant son origine dans le ventricule.

*Stimulation A* : stimulation délivrée à l'oreillette.

*Stimulation V :* stimulation délivrée au ventricule.

*Événement auriculaire :* détection P ou bien stimulation A ;

*Cycle cardiaque :* intervalle de temps séparant deux événements de même nature dans la même cavité, par exemple séparant deux détections P, ou deux stimulations A.

*Délai PR (ou délai AR) :* délai de conduction auriculo-ventriculaire, c'est-à-dire intervalle de temps séparant une dépolarisation auriculaire spontanée (P) ou stimulée (A) de la dépolarisation ventriculaire spontanée (R) consécutive induite.

Au départ, le fonctionnement du stimulateur est un fonctionnement en mode AAI avec surveillance de l'activité ventriculaire : l'algorithme recherche la présence ou l'absence d'une activité ventriculaire, qui dans ce dernier cas pourrait laisser suspecter un BAV, de manière à éventuellement basculer en mode DDD de stimulation double chambre avec association auriculo-ventriculaire, c'est-à-dire avec calcul et application d'un délai auriculo-ventriculaire pour la stimulation contrôlée du ventricule.

Dans ce mode AAI, l'absence d'activité ventriculaire est acceptée sur un nombre donné de cycles sans qu'une stimulation ventriculaire ne soit déclenchée. Ce nombre est programmable, par exemple 2 cycles.

Comme exposé plus haut, quatre situations peuvent induire le passage au mode DDD :

1 °) un bloc auriculo-ventriculaire du premier degré (BAV1 ), qui se manifeste par un délai de conduction auriculo-ventriculaire supérieur à une valeur donnée, typiquement 350 ms après une détection P ou 450 ms après une stimulation A. La commutation en mode DDD intervient, typiquement, après six cycles consécutifs ;

2°) un bloc auriculo-ventriculaire du second degré (BAV2), qui se manifeste par un rapport : nombre d'ondes P non extrasystoliques/nombre d'ondes R non extrasystoliques, supérieur à 1. La commutation intervient si, typiquement, l'appareil détecte un rapport supérieur ou égal à 12/9 (3 ondes P bloquées sur les 12 derniers cycles);

3°) un bloc auriculo-ventriculaire complet (BAV3), qui se manifeste par des ondes auriculaires (stimulées ou spontanées) bloquées, c'est-à-dire des dépolarisations auriculaires non suivies de dépolarisations ventriculaires. Typiquement, la commutation intervient si l'appareil détecte une succession de deux ondes auriculaires bloquées (détectées ou stimulées)

4°) une pause ventriculaire, qui se manifeste par plus de trois secondes écoulées sans détection ventriculaire, indépendamment du point de savoir si cette pause résulte, ou non, d'un trouble de la conduction auriculo-ventriculaire.

En présence de l'un de ces critères, le dispositif bascule du mode AAI au mode DDD.

Après retour d'une activité ventriculaire spontanée sur un certain nombre de cycles, par exemple après 12 détections R, ou après un nombre prédéterminé de cycles en mode DDD, par exemple 100 cycles avec stimulation V, le dispositif retourne alors de DDD en AAI et demeure en mode AAI tant qu'aucun des trois critères précités de commutation de AAI vers DDD n'est vérifié.

Par ailleurs, pour limiter le nombre de commutations successives de AAI vers DDD sur une période donnée, le dispositif bascule définitivement en mode DDD, avec des paramètre préprogrammés, si par exemple :

- le nombre de commutations AAI → DDD sur une période de 24 heures consécutives excède un nombre maximal autorisé, programmable, par exemple 15 commutations, ou
- pendant trois jours consécutifs, le nombre quotidien de commutations AAI → DDD excède un nombre maximal autorisé, programmable, par exemple 5 commutations par 24 h.

Ce mode de fonctionnement connu est aménagé selon l'invention de la manière que l'on va exposer.

*Détection des troubles du rythme auriculaire et autres états particuliers*

Un premier perfectionnement consiste à détecter l'apparition ou le risque d'apparition ("suspicion") des troubles du rythme auriculaire (TdRA).

En effet, un TdRA se caractérise par la présence de plusieurs dépolarisations auriculaires au cours d'un même cycle, ce qui a pour effet de fausser les critères de détection de BAV1, BAV2 et BAV3 ci-dessus, basés sur l'analyse de la conduction auriculo-ventriculaire ; le critère de pause ventriculaire, qui ne fait pas intervenir la survenue d'un événement auriculaire, reste en revanche pertinent.

Les TdRA peuvent être détectés par un algorithme tel que celui décrit dans le EP-A-0 755 696 (ELA Medical) , qui explique notamment la manière de discriminer entre extrasystoles auriculaires (ESA) isolées et

TdRA proprement dits par une détection en deux temps : phase de suspicion de TdRA, suivie d'une phase de confirmation de TdRA.

Dans le cadre de la présente invention, la détection selon les critères précités de BAV1, BAV2 et BAV3 est suspendue dès que l'algorithme entre en phase de suspicion de TdRA. Pendant toute la phase de suspicion, seul le critère de pause ventriculaire est susceptible de provoquer une commutation du dispositif en mode DDD.

En cas de TdRA confirmé, l'appareil commute en mode DDD de repli. Si, en revanche, la phase de suspicion de TdRA ne débouche pas sur une confirmation de TdRA (c'est-à-dire que, vraisemblablement, il y a simplement eu ESA isolée), les critères de détection de BAV1, BAV2 et BAV3 sont rétablis, de préférence après quelques cycles, c'est-à-dire que les critères de détection de BAV1, BAV2 et BAV3 seront suspendus pendant toute la phase de suspicion jusqu'à N cycles après la fin de suspicion de TdRA.

Les enseignements de l'invention sont également applicables à des états particuliers, autres que les TdRA, susceptibles d'être diagnostiqués par le dispositif, notamment les phases de décompensation cardiaque, où le mauvais état hémodynamique passager du patient est détecté par une mesure d'impédance intracardiaque ou par une mesure d'accélération intracavitaire opérée par un capteur intégré à la sonde.

Il peut également s'agir d'une phase d'arythmie diagnostiquée par un algorithme dédié d'analyse de rythme cardiaque, par exemple l'algorithme décrit dans le EP-A-0 838 235 (ELA Medical).

*Gestion différenciée de la commutation de AAI en DDD*

Un deuxième perfectionnement consiste à commander de façon différenciée la commutation en mode DDD, d'une part en fonction du type de trouble rencontré (BAV1, BAV2, BAV3 ou pause), et d'autre part en fonction des circonstances de survenue de ce trouble, c'est-à-dire en fonction de l'état du patient (effort, sommeil ou autre).

En effet, les commutations en DDD sur certains types de BAV, ou dans certaines circonstances, peuvent ne pas être souhaitées.

Tel est le cas d'un patient implanté pour BAV1, où le dispositif basculerait rapidement, puis définitivement, en mode DDD - de façon inappropriée compte tenu de la moindre gravité du BAV1 et de son caractère souvent sporadique.

De même, un patient présentant une conduction normale au repos mais un BAV1 asymptomatique à l'effort ou en sommeil verrait le dispositif commuter régulièrement en DDD lors de ces périodes avec à terme, si ces commutations sont trop nombreuses, une commutation définitive en mode DDD - alors que le patient aurait pu bénéficier du mode AAI au moins pendant les périodes de repos.

Pour tenir compte de ces circonstances, le dispositif de l'invention comprend des moyens détecteurs d'effort et/ou de sommeil.

La détection d'un effort peut être réalisée, de manière en elle-même connue, au moyen d'un capteur d'activité, typiquement un accéléromètre, permettant de détecter rapidement un changement de l'activité du porteur de l'appareil, comme l'enseigne par exemple le EP-A-0 550 293 (ELA Médical), ou au moyen d'une combinaison de capteurs physiologique et d'activité, comme l'enseigne par exemple le EP-A-0 750 920 (ELA Médical) précité.

Quant à la détection du sommeil, elle peut être réalisée par diverses techniques, en elles-mêmes connues, de discrimination entre éveil et sommeil. La technique la plus simple consiste à utiliser une horloge interne du dispositif, commutant un indicateur à heures fixes. Il est également possible, comme enseigné par le EP-A-0 719 568 (Ela Médical), d'opérer la discrimination entre éveil et sommeil par analyse d'un signal physiologique de ventilation-minute (MV) : en effet, la variation circadienne de la fréquence et de l'amplitude des cycles respiratoires successifs du patient est bien reproduite par le signal MV ; un calcul de la ventilation moyenne sur 24 h permet donc d'opérer une discrimination satisfaisante entre une ventilation d'éveil et une ventilation de sommeil. On peut également utiliser un capteur d'activité, typiquement un accéléromètre ("capteur G"), dont le signal permet de détecter les mouvements du patient ; l'information de ce type de capteur n'est en elle-même pas très spécifique des phases d'éveil et de sommeil, mais on sait combiner les signaux délivrés par un capteur G et un capteur MV pour en déduire des informations signifiantes, comme cela est décrit par exemple dans les EP-A-0 750 920 (Ela Médical) et EP-A-0 770 407 (Ela Médical), auxquels on pourra se référer pour de plus amples détails.

Par ailleurs, pour permettre une commutation différenciée selon le trouble détecté et les circonstances de survenue du trouble, le dispositif comporte une grille de compteurs où chaque type de trouble (BAV1, BAV2, BAV3 ou pause) est associé à une circonstance de survenue (effort, sommeil ou autre), soit douze critères avec chacun un compteur incrémentable indépendamment.

Les commutations AAI → DDD sont alors programmées séparément sur chacun de ces douze critères, en interdisant ou autorisant le basculement en DDD pour chacun des douze critères ; d'autre part, lorsque le basculement en DDD est autorisé, le seuil de déclenchement du basculement (franchissement du seuil par le compteur associé) peut être différent pour chaque critère.

De préférence, le dispositif ne commute pas en mode DDD sur détection d'un BAV1 (et ce, quel que soit l'état du patient) ; il ne commute pas non plus en DDD en cas de BAV2 nocturne. En revanche, la commutation DDD est autorisée pour tout BAV2 hors sommeil, pour tout BAV3 ou pour toute pause.

Ces conditions peuvent être résumées par la table de vérité suivante :

Tableau 1

|         | BAV1 | BAV2   | BAV3   | pause   |
|---------|------|--------|--------|---------|
| effort  | Non  | → DDD  | → DDD  | → DDD   |
| sommeil | Non  | Non    | → DDD  | → DDD   |
| autre   | Non  | → DDD  | → DDD  | → DDD   |

*Gestion différenciée de la commutation inverse de DDD en AAI*

Un troisième perfectionnement consiste, lorsque le dispositif a commuté en mode DDD, à modifier la gestion de la commutation inverse de retour en mode AAI pour des états de patient particuliers, et ce pendant la durée où ces états restent présents.

Ces états particuliers peuvent être, comme précédemment, l'effort ou le sommeil (les enseignements de l'invention étant également applicables à d'autres états particuliers, notamment les phases de décompensation cardiaque ou les phases d'arythmie).

Ainsi, lorsqu'une commutation AAI → DDD a lieu à l'effort, l'invention propose d'inhiber le retour au mode AAI ou d'en modifier temporairement les critères dans un sens plus restrictif, tant que dure l'effort.

En effet, s'il y a eu par exemple des commutations à l'effort, on peut penser que le BAV va persister jusqu'à la fin de l'effort et les tentatives de recommutation en AAI tant que cet effort persiste ne sont pas appropriées, car elles risquent d'entraîner des pauses symptomatiques pendant cet effort.

L'effort terminé, il est par contre souhaitable de pouvoir au plus vite retrouver un fonctionnement AAI (puisque la conduction est a priori redevenue normale) et donc de forcer la recommutation vers le mode AAI Inversement, une fois que la fin d'effort aura été détectée, l'invention propose de forcer la recommutation au mode AAI dès détection de cette fin d'effort, ou de faciliter cette recommutation en modifiant temporairement les critères de recommutation dans un sens extensif.

Plus précisément, par exemple sur une commutation AAI → DDD survenant sur détection d'un BAV1 ou BAV2 à l'effort, ou après un nombre prédéterminé de commutations AAI → DDD sur BAV1 ou BAV2 pendant un effort, les critères de retour en mode AAI peuvent être modifiés de la manière suivante :

- retour en AAI suspendu jusqu'à la fin de l'effort (c'est-à-dire que l'on reste en mode DDD tant que dure cet effort), ou bien
- retour en AAI autorisé sur un critère restrictif, par exemple 30 cycles consécutifs - au lieu de 12 - avec détection R ;

Inversement, lorsque la fin d'effort est détectée, les critères de retour au mode AAI peuvent être modifiés, par exemple, de la manière suivante :

- retour immédiat et inconditionnel au mode AAI, ou bien
- retour au mode AAI autorisé en présence de 6 cycles consécutifs - au lieu de 12 - avec détection R, ou après seulement 10 cycles - au lieu de 100 - avec stimulation ventriculaire.

Les critères ci-dessus peuvent s'appliquer de manière indépendante ou bien conjointe par rapport aux critères de commutation à l'effort sur détection de BAV1, BAV2, BAV3 ou de pause.

*Commutation durable en mode DDD*

Un quatrième perfectionnement consiste à contrôler la commutation définitive de manière à tenir compte de l'état du patient et des circonstances ayant entouré les commutations déjà intervenues.

On entendra par "commutation durable" un état dans lequel l'algorithme de pilotage inhibe le retour du mode DDD au mode AAI, et force donc le mode de fonctionnement au mode DDD, au moins pendant une durée minimale prédéterminée - donc en laissant au dispositif une possibilité de retour éventuel automatique au mode AAI.

Cette commutation durable pourra être ultérieurement transformée en une "commutation définitive", où le retour du mode DDD au mode AAI sera interdit, le mode DDD étant alors imposé jusqu'à reprogrammation du dispositif, sans possibilité de retour automatique au mode AAI.

Le dispositif comprend, outre des moyens de détecter l'effort ou le sommeil (voir ci-dessus), une grille de compteurs de commutation affectés aux quatre critères ayant pu déclencher un basculement du mode AAI vers le mode DDD (BAV1, BAV2, BAV3 ou pause) et aux états susceptibles d'être diagnostiqués (effort, sommeil ou autre).

Le dispositif déclenche une commutation durable en mode DDD lorsque les compteurs atteignent une configuration de comptage prédéterminée.

L'ensemble étant programmable, le praticien dispose d'une très grande liberté pour gérer les conditions de la commutation durable.

Il est notamment souhaitable d'empêcher une commutation durable dans certains cas de figure, c'est-à-dire que l'incrémentation du (des) compteur(s) correspondants) ne pourra pas provoquer de commutation durable.

Tel est le cas notamment des commutations à l'effort ou en sommeil : il peut s'agir des commutations sur BAV1 seulement, ou des commutations sur BAV2 et BAV3, ou des commutations sur toute combinaison particulière des critères de BAV. Les commutations sur critères de pause seront en revanche prises en compte pour permettre une commutation durable en mode DDD.

De même, il est souhaitable de ne pas prendre en compte les commutations non isolées, une commutation non isolée étant définie comme une commutation séparée de la précédente commutation d'un nombre de cycles en AAI inférieur à un nombre donné, typiquement une commutation séparée de la précédente par moins de 100 cycles en mode AAI.

Le dispositif opère une commutation durable en mode DDD si, par exemple, le nombre de commutations effectivement prises en compte par les compteurs ci-dessus est supérieur à un seuil sur une période donnée, par exemple plus de quinze commutations par 24 heures, ou plus de cinq commutations par 24 heures sur trois jours consécutifs.

Il est également possible de prendre en compte comme critère de commutation durable le pourcentage d'évènements ventriculaires stimulés sur une période donnée (24 heures) : si ce pourcentage dépasse un certain seuil, l'appareil déclenche une commutation durable en mode DDD même si les compteurs n'ont pas encore atteint la configuration de comptage qui, sinon, aurait déclenché cette commutation durable.

*Commutation définitive en mode DDD*

Un cinquième perfectionnement de l'invention consiste à commander une commutation non plus durable, mais définitive, vers le mode DDD.

À cet effet, après une ou plusieurs commutations durables le dispositif examine s'il y a, ou non, lieu de transformer cette commutation durable en une commutation définitive.

Par exemple, à chaque fin de phase de sommeil le dispositif examine si le mode courant est un mode DDD suite à une commutation durable. Si tel est le cas, le dispositif repasse en mode AAI, en réinitialisant tous les compteurs de commutation durable. Si ce phénomène se répète N jours consécutifs (ou si le pourcentage d'évènements ventriculaires stimulés est supérieur à un seuil donné sur N jours consécutifs), alors le dispositif bascule définitivement en DDD, et cette commutation définitive ne pourra être levée que par une reprogrammation de l'appareil par un praticien.

*Gestion particulière des commutations à l'effort*

Un sixième perfectionnement de l'invention consiste à remplacer les durées de références fixes typiques de 350 ou 450 ms (délai PR ou AR de référence pour la détection d'un BAV1) par une durée ajustable en fonction de l'état du patient, plus précisément une durée qui puisse s'adapter à l'effort.

En effet, en cas d'effort, le rythme cardiaque s'accroît et les valeurs fixes typiques de 350 ou 450 ms se révèlent trop longues, car il n'est pas souhaitable, sur un plan hémodynamique, de laisser l'intervalle PR ou AR s'allonger jusqu'à 350 ou 450 ms avant de commuter au mode DDD.

Pour cela, l'invention propose d'utiliser pour l'intervalle PR de référence une valeur PR(f) adaptable à l'effort, décroissante en fonction de la fréquence cardiaque f.

On peut par exemple prévoir pour l'intervalle PR de référence une loi de variation linéaire sur un intervalle délimité par deux fréquences $f_{inf}$ et $f_{sup}$, conformément à la relation suivante :

$$PR(f) = PR(f_{inf}) - [ (f - f_{inf}) \times [(PR(f_{inf}) - PR(f_{sup})] / (f_{sup} - f_{inf}) ],$$

avec par exemple $PR(f_{inf})$ = 350 ms et $PR(f_{sup})$ = 250 ms.

On peut choisir pour $f_{inf}$ et $f_{sup}$, respectivement, la fréquence de base de stimulation et la fréquence maximale de stimulation programmée pour l'appareil, par exemple $f_{inf} = f_{base}$ = 30 cpm et $f_{sup} = f_{max}$ = 83 cpm.

En variante, il est également possible de choisir pour $f_{inf}$ et $f_{sup}$ des fréquences fixes, par exemple $f_{inf}$ = 60 cpm et $f_{sup}$ = 150 cpm. Dans ce cas, pour $f < f_{inf}$, la valeur de l'intervalle de référence sera constant (typiquement PR = $PR_{inf}$ = 350 ms), de même pour $f > f_{sup}$ (typiquement $PR_{sup}$ = 250 ms) avec une variation linéaire continue entre ces deux valeurs.

La formule ci-dessus peut être transposée au calcul de l'intervalle AR de référence, avec AR(f) = PR(f) + x ms (x pouvant être une valeur constante, par exemple 100 ms, ou une valeur variable, fonction de la fréquence cardiaque).

*Documentation de la conduction auriculo-ventriculaire*

Un septième perfectionnement consiste à monitorer de façon différenciée la conduction auriculo-ventriculaire spontanée au cours du nycthémère et en fonction de l'activité : dans la mesure où l'on dispose de toutes les informations nécessaires grâce à la mise en oeuvre des perfectionnements précités, il est effet intéressant de pouvoir par exemple monitorer les intervalles PR et AR en fonction de l'heure de la journée, de la fréquence auriculaire et en fonction de l'état éveil/sommeil ou repos/effort du patient, et ce sur une période de durée variable (24 heures, 1 semaine, 1 mois, 3 mois ou 6 mois).

Les durées des intervalles PR et AR dans les différents états (effort, repos ou sommeil) sont alors mémorisés de manière différenciée, et des histogrammes sont construits sur la période concernée, par exemple des histogrammes des intervalles PR et AR en fonction du nycthémère.

Les épisodes de trouble de la conduction sont mémorisés sous forme d'histogrammes. Ces histogrammes permettent d'apporter une connaissance sur la durée des BAV paroxystiques, qui est inconnue.

**Revendications**

1. Un dispositif médical implantable actif, notamment un stimulateur cardiaque, défibrillateur et/ou cardioverteur, comprenant :

- des moyens de détection d'événements auriculaires et ventriculaires spontanés,
- des moyens de stimulation ventriculaire et auriculaire,
- des moyens aptes à faire fonctionner le dispositif en mode AAI avec détection ventriculaire,
- des moyens aptes à faire fonctionner le dispositif en mode DDD, et
- des moyens de détection d'état, aptes à diagnostiquer au moins un état spécifique temporaire du patient porteur du dispositif, ces moyens étant aptes à diagnostiquer :

. au moins une phase d'effort et/ou une phase de sommeil du patient, et
.une phase de suspicion de trouble du rythme auriculaire du patient,

- des moyens de commutation de mode, aptes à commander en fonction de critères prédéterminés le basculement du mode AAi en mode DDD, et inversement le retour du mode DDD au mode AAI, lesdits critères prédéterminés comprenant :

■ des critères de basculement du mode AAI vers le mode DDD, aptes à commander le basculement lorsqu'est vérifiée l'une au moins des conditions suivantes :

a) le nombre d'événements auriculaires suivis d'une détection ventriculaire survenant après un délai supérieur à une première durée de référence excède un premier nombre autorisé ;
b) le nombre d'événements auriculaires non suivis d'une détection ventriculaire excède un deuxième nombre autorisé parmi un troisième nombre donné d'événements auriculaires consécutifs ;
c) le nombre d'événements auriculaires consécutifs non suivis d'une détection ventriculaire excède un quatrième nombre autorisé ; et
d) l'intervalle séparant deux événements ventriculaires excède une deuxième durée de référence ; et dispositif dans lequel :

- les moyens de commutation de mode sont des moyens opérant en réponse aux moyens de détection d'état ladite phase d'effort et/ou ladite phase de sommeil du patient pour modifier lesdits critères de basculement du mode AAI vers le mode DDD pendant la durée où aptes à diagnostiquer au moins une phase d'effort et/ou une phase de sommeil du patient est diagnostiquée,

ce dispositif étant **caractérisé en ce que** :

- les moyens de commutation de mode sont des moyens aptes à suspendre, pendant une durée au moins égale à la durée de la phase de suspicion de trouble du rythme auriculaire du patient, la vérification des conditions a) à c) des critères de basculement du mode AAI vers le mode DDD, tout en maintenant la vérification de la condition d).

2. Le dispositif de la revendication 1, dans lequel lesdits moyens de détection d'état sont des moyens aptes à diagnostiquer en outre une phase de décompensation cardiaque du patient.

3. Le dispositif de la revendication 1, dans lequel les moyens de commutation de mode comprennent une grille de compteurs affectés respectivement aux conditions a) à d) des critères de basculement du mode AAI vers le mode DDD et aux types d'états susceptibles d'être diagnostiqués, le compteur correspondant étant incrémenté lorsque la condition respective est vérifiée et que le type d'état respectif est détecté.

4. Le dispositif de la revendication 3, dans lequel les moyens de commutation de mode comprennent une grille de seuils homologue de la grille de compteurs, chaque seuil étant programmable, et sont aptes à forcer le basculement du mode AAI vers le mode DDD lorsque l'un au moins des seuils est atteint par le compteur correspondant.

**5.** Le dispositif de la revendication 1, dans lequel les moyens de commutation de mode sont des moyens opérant en réponse aux moyens de détection d'état pour modifier lesdits critères de retour au mode AAI après basculement vers le mode DDD pendant le temps où une phase d'effort ou de sommeil est diagnostiquée.

**6.** Le dispositif de la revendication 5, dans lequel les moyens de commutation de mode sont des moyens opérant en réponse aux moyens de détection d'état pour suspendre temporairement tout retour au mode AAI après basculement vers le mode DDD pendant le temps où une phase d'effort ou de sommeil est diagnostiquée.

**7.** Le dispositif de la revendication 5, dans lequel les moyens de commutation de mode sont des moyens opérant en réponse aux moyens de détection d'état pour modifier temporairement dans un sens restrictif lesdits critères de retour au mode AAI après basculement vers le mode DDD pendant le temps où la phase d'effort ou de sommeil est diagnostiquée.

**8.** Le dispositif de la revendication 7, dans lequel les moyens de commutation de mode sont des moyens opérant en réponse aux moyens de détection d'état pour commander sans délai le retour au mode AAI après basculement vers le mode DDD après la fin de ladite phase d'effort ou de sommeil.

**9.** Le dispositif de la revendication 7, dans lequel les moyens de commutation de mode sont des moyens opérant en réponse aux moyens de détection d'état pour modifier temporairement dans un sens extensif lesdits critères de retour au mode AAI après basculement vers le mode DDD après la fin de ladite phase d'effort ou de sommeil.

**10.** Le dispositif de la revendication 1, dans lequel:

- lesdits critères prédéterminés comprennent également :

■ des critères de commutation durable en mode DDD, et

- les moyens de commutation de mode comprennent également des moyens de commutation durable, aptes à inhiber le retour du mode DDD au mode AAI et à forcer le mode de fonctionnement au mode DDD en fonction desdits critères de commutation durable.

**11.** Le dispositif de la revendication 10, dans lequel les moyens de commutation durable comprennent des compteurs incrémentés sélectivement en fonction des conditions a) à d) des critères de basculement du mode AAI vers le mode DDD lorsque la condition respective est vérifiée, et des moyens pour inhiber le retour du mode DDD au mode AAI et forcer le mode de fonctionnement au mode DDD lorsque les compteurs atteignent une configuration de comptage prédéterminée.

**12.** Le dispositif de la revendication 11, dans lequel les moyens de commutation durable sont aptes à inhiber l'incrémentation des compteurs lorsque l'une des conditions a) à c) est vérifiée tout en autorisant l'incrémentation lorsque la condition d) est vérifiée, en cas de commutation consécutive à un diagnostic de phase d'effort ou de sommeil.

**13.** Le dispositif de la revendication 11, dans lequel les moyens de commutation durable sont aptes à inhiber rincrémentation des compteurs en cas de survenue d'une commutation séparée de la commutation précédente par un nombre de cycles en mode AAI inférieur à un cinquième nombre prédéterminé.

**14.** Le dispositif de la revendication 10, dans lequel les moyens de commutation durable sont aptes à inhiber le retour du mode DDD au mode AAI et forcer le mode de fonctionnement au mode DDD lorsque le nombre de commutations du mode AAI vers le mode DDD est supérieur à un sixième nombre prédéterminé sur une première période temporelle prédéterminée.

**15.** Le dispositif de la revendication 10, dans lequel les moyens de commutation durable sont aptes à inhiber le retour du mode DDD au mode AAI et forcer le mode de fonctionnement au mode DDD lorsque le pourcentage d'événements ventriculaires stimulés sur une deuxième période temporelle prédéterminée est supérieur à un premier seuil prédéterminé.

**16.** Le dispositif de la revendication 10, dans lequel les moyens de commutation durable sont des moyens aptes à commander le retour au mode de fonctionnement en mode AAI à la fin de chaque phase de sommeil.

**17.** Le dispositif de la revendication 11, dans lequel les moyens de commutation durable sont des moyens aptes à réinitialiser lesdits compteurs à la fin de chaque phase de sommeil.

**18.** Le dispositif de la revendication 10, dans lequel :

- lesdits critères prédéterminés comprennent également :

■ des critères de commutation définitive en mode DDD, et

- les moyens de commutation de mode comprennent également des moyens de commutation définitive, aptes à interdire le retour du mode DDD au mode AAI et à imposer un mode de fonctionnement DDD jusqu'à reprogrammation du dispositif, en fonction desdits critères de commutation définitive.

**19.** Le dispositif de la revendication 18, dans lequel les moyens de commutation définitive sont aptes à interdire le retour du mode DDD au mode AAI et imposer un mode de fonctionnement DDD jusqu'à reprogrammation du dispositif lorsque le nombre de commutations durables du mode AAI vers le mode DDD est supérieur à un septième nombre prédéterminé sur une troisième période temporelle prédéterminée.

**20.** Le dispositif de la revendication 18, dans lequel les moyens de commutation définitive sont aptes à interdire le retour du mode DDD au mode AAI et imposer un mode de fonctionnement DDD jusqu'à reprogrammation du dispositif lorsque le pourcentage d'événements ventriculaires stimulés sur une quatrième période temporelle prédéterminée est supérieur à un deuxième seuil prédéterminé.

**21.** Le dispositif de la revendication 1, dans lequel les moyens de commutation de mode réduisent ladite première durée de référence pendant le temps où une phase d'effort est diagnostiquée.

**22.** Le dispositif de la revendication 21, dans lequel les moyens de commutation de mode réduisent ladite première durée de référence à une valeur qui est une fonction inverse de la fréquence de stimulation.

**23.** Le dispositif de la revendication 22, dans lequel ladite fonction inverse est une fonction monotone décroissante depuis la fréquence de base de stimulation jusqu'à la fréquence maximale de stimulation.

**24.** Le dispositif de la revendication 22, dans lequel ladite fonction inverse est une fonction monotone décroissante depuis une fréquence basse, supérieure à la fréquence de base de stimulation, jusqu'à une fréquence haute, supérieure à la fréquence maximale de stimulation, et une fonction constante au-dessous de la fréquence de base de stimulation et au-dessus de la fréquence maximale de stimulation.

**25.** Le dispositif de la revendication 1, comprenant en outre :

- des moyens d'enregistrement différencié de données cardiaques, aptes à mémoriser spécifiquement certaines données prédéterminées pendant le temps où ledit état spécifique, temporaire, est diagnostiqué.

**26.** Le dispositif de la revendication 25, dans lequel lesdites données prédéterminées mémorisées par les moyens d'enregistrement différencié comprennent les valeurs des durées de conduction auriculo-vertriculaire.

**27.** Le dispositif de la revendication 25, dans lequel lesdites données prédéterminées mémorisées par les moyens d'enregistrement différencié comprennent la durée d'épisodes au cours desquels se succèdent des commutations toutes séparées les unes des autres d'un nombre de cycles en mode AAI inférieur à un cinquième nombre prédéterminé.

**Claims**

**1.** An active implantable medical device, such as a cardiac pacemaker, defibrillator and/or cardioverter, comprising:

- means for detecting spontaneous atrial and ventricular events,
- means for ventricular and atrial stimulation,
- means able to operate the device in AAI mode with ventricular detection,

- means able to operate the device in DDD mode, and
- state detection means able to diagnose at least one temporary specific state of the patient carrying the device, these means being able to diagnose:

· at least one effort phase and/or sleep phase of the patient, and
· a phase of suspicion of disorder of the patient's atrial rhythm,

- means for mode switching, able to order the switching from AAI mode into DDD mode as a function of predetermined criteria, and inversely the return from DDD mode to AAI mode,

said predetermined criteria comprising:

■ criteria for switching from AAI mode to DDD mode, able to order the switching when at least one of the following conditions is verified:

a) the number of atrial events followed by a ventricular detection occurring after a time-limit longer than a first reference duration exceeds a first authorised number;
b) the number of atrial events not followed by a ventricular detection exceeds a second authorised number among a third given number of consecutive atrial events;
c) the number of consecutive atrial events not followed by a ventricular detection exceeds a fourth authorised number; and
d) the interval separating two ventricular events exceeds a second reference duration; and
device, wherein:

- the mode switching means are means operating in response to the state detection means able to diagnose at least an effort phase and/or a sleep phase of the patient to modify said criteria for switching from AAI mode to DDD mode for the length of time when said effort phase and/or said sleep phase of the patient is diagnosed,

2. The device of claim 1, wherein said state detection means are means furthermore able to diagnose a phase of cardiac decompensation of the patient.

3. The device of claim 1, wherein the mode switching means include an array of counters assigned respectively to the conditions a) to d) of the criteria for switching from AAI mode to DDD mode and to the types of states likely to be diagnosed, the corresponding counter being incremented when the respective condition is verified and the respective state type is detected.

4. The device of claim 3, wherein the mode switching means comprise an array of thresholds homologous with the counter array, each threshold being programmable, and are able to force the switching from AAI mode to DDD mode when at least one of the thresholds is reached by the corresponding counter.

5. The device of claim 1, wherein the mode switching means are means operating in response to the state detection means to modify said criteria of return to AAI mode after switching to DDD mode during the time when an effort or sleep phase is diagnosed.

6. The device of claim 5, wherein the mode switching means are means operating in response to the state detection means to temporarily suspend any return to AAI mode after switching to DDD mode during the time when an effort or sleep phase is diagnosed.

7. The device of claim 5, wherein the mode switching means are means operating in response to the state detection means to temporarily modify in a restrictive direction said criteria of return to AAI mode after switching to DDD mode during the time when the effort or sleep phase is diagnosed.

8. The device of claim 7, wherein the mode switching means are means operating in response to the state detection means to order without delay the return to AAI mode after switching to DDD mode after the end of said effort or sleep phase.

9. The device of claim 7, wherein the mode switching means are means operating in response to the state detection means to temporarily modify in an extensive direction said criteria of return to AAI mode after switching to DDD mode after the end of said effort or sleep phase.

10. The device of claim 1, wherein:

   - said predetermined criteria also comprise:

      ■ criteria of durable switching to DDD mode; and

   - the mode switching means also comprise durable switching means, able to inhibit the return from DDD mode to AAI mode and to force the operating mode to DDD mode as a function of said criteria of durable switching.

11. The device of claim 10, wherein the durable switching means comprise counters incremented selectively as a function of the conditions a) to d) of the criteria for switching from AAI mode to DDD mode when the respective condition is verified, and means to inhibit the return from DDD mode to AAI mode and to force the operating mode to DDD mode when the counters reach a predetermined counting configuration.

12. The device of claim 11, wherein the durable switching means are able to inhibit the incrementing of the counters when one of the conditions a) to c) is verified, while authorizing the incrementing when the condition d) is verified, in the event of switching consecutive to a diagnosis of an effort or sleep phase.

13. The device of claim 11, wherein the durable switching means are able to inhibit the incrementing of the counters in the event of the occurrence of a switching separated from the preceding switching by a number of cycles in AAI mode lower than a fifth predetermined number.

14. The device of claim 10, wherein the durable switching means are able to inhibit the return from DDD mode to AAI mode and to force the operating mode to DDD mode when the number of switchings from AAI mode to DDD mode is higher than a sixth predetermined number over a first predetermined time period.

15. The device of claim 10, wherein the durable switching means are able to inhibit the return from DDD mode to AAI mode and to force the operating mode to DDD mode when the percentage of stimulated ventricular events over a second predetermined time period is higher than a first predetermined threshold.

16. The device of claim 10, wherein the durable switching means are means able to order the return to the operating mode in AAI mode at the end of each sleep phase.

17. The device of claim 11, wherein the durable switching means are means able to reinitialise said counters at the end of each sleep phase.

18. The device of claim 10, wherein:

   - said predetermined criteria further comprise:

      ■ criteria of final switching into DDD mode, and

   - the mode switching means further comprise final switching means able to prohibit the return from DDD mode to AAI mode and to impose a DDD operating mode until reprogramming of the device, as a function of said criteria of final switching.

19. The device of claim 18, wherein the final switching means are able to prohibit the return from DDD mode to AAI mode and to impose a DDD operating mode until reprogramming of the device when the number of durable switchings from AAI mode to DDD mode is higher than a seventh predetermined number over a third predetermined time period.

20. The device of claim 18, wherein the final switching means are able to prohibit the return from DDD mode to AAI mode and to impose a DDD operating mode until reprogramming of the device when the percentage of stimulated ventricular events over a fourth predetermined time period is greater than a second predetermined threshold.

21. The device of claim 1, wherein the mode switching means reduce said first reference duration during the time when an effort phase is diagnosed.

22. The device of claim 21, wherein the mode switching means reduce said first reference duration to a value which is a reciprocal function of the stimulation frequency.

23. The device of claim 22, wherein said reciprocal function is a decreasing monotonic function from the base stimulation frequency to the maximum stimulation frequency.

24. The device of claim 22, wherein said reciprocal function is a decreasing monotonic function from a low frequency, higher than the base stimulation frequency, to a high frequency, higher than the maximum stimulation frequency, and a constant function below the base stimulation frequency and above the maximum stimulation frequency.

25. The device of claim 1, further comprising:

      - means for differentiated recording of cardiac data, able to specifically memorize certain predetermined data during the time when said specific temporary state is diagnosed.

26. The device of claim 25, wherein said predetermined data memorized by the differentiated recording means includes the values of the durations of atrio-ventricular conduction.

27. The device of claim 25, wherein said predetermined data memorized by the differentiated recording means includes the duration of episodes during which switchings follow one another that are all separated from each other by a number of cycles in AAI mode lower than a fifth predetermined number.

**Patentansprüche**

1. Aktive implantierbare medizinische Vorrichtung, insbesondere Herzschrittmacher, Defibrillator und/oder Kardioverter, mit:

      - Mitteln zur Erfassung spontaner Herzvorhof- und Herzkammerereignisse,
      - Mitteln zur Herzkammer- und Herzvorhofstimulation,
      - Mitteln, die geeignet sind, die Vorrichtung im AAI-Modus mit Herzkammererfassung zu betreiben,
      - Mitteln, die geeignet sind, die Vorrichtung im DDD-Modus zu betreiben, und
      - Mitteln zur Zustandserfassung, die geeignet sind, zumindest einen spezifischen temporären Zustand des die Vorrichtung tragenden Patienten zu diagnostizieren, wobei diese Mittel geeignet sind:

            · mindestens eine Anstrengungsphase und/oder eine Schlafphase des Patienten, und
            · eine Phase der Vermutung einer Störung des Herzvorhof-Rhythmus des Patienten,

      zu diagnostizieren,
      - Mitteln zur Modusumschaltung, die geeignet sind, in Abhängigkeit vorbestimmter Kriterien das Umschalten vom AAI-Modus in den DDD-Modus anzuordnen, und umgekehrt die Rückkehr vom DDD-Modus zum AAI-Modus,

   wobei die vorbestimmten Kriterien folgendes umfassen:

      ■ Kriterien zur Umschaltung vom AAI-Modus zum DDD-Modus, die geeignet sind, die Umschaltung anzuordnen, wenn zumindest eine der folgenden Bedingungen bestätigt ist:

            a) die Anzahl an Herzvorhofereignissen, die von einer Erfassung in der Herzkammer gefolgt werden, welche nach einer Frist erfolgt, die länger als eine erste Bezugsdauer ist, übersteigt eine erste zulässige Zahl;
            b) die Anzahl an Herzvorhofereignissen, die nicht von einer Erfassung in der Herzkammer gefolgt werden, übersteigt eine zweite zulässige Zahl innerhalb einer dritten gegebenen Anzahl an aufeinander folgenden Herzvorhofereignissen;
            c) die Anzahl an aufeinander folgenden Herzvorhofereignissen, die nicht von einer Erfassung in der Herzkammer gefolgt werden, übersteigt eine vierte zulässige Zahl; und
            d) das zwei Herzkammerereignisse trennende Intervall übersteigt eine zweite Bezugsdauer; und

Vorrichtung bei welcher:

- die Mittel zur Modusumschaltung Mittel sind, die in Antwort auf die Mittel zur Zustandserfassung, die zur Diagnose mindestens einer Anstrengungsphase und/oder einer Schlafphase des Patienten geeignet sind, arbeiten, um die Kriterien zur Umschaltung vom AAI-Modus zum DDD-Modus während der Dauer zu modifizieren, wo die Anstrengungsphase und/oder Schlafphase des Patienten diagnostiziert wird,

wobei die Vorrichtung **dadurch gekennzeichnet ist, dass**:

- die Mittel zur Modusumschaltung Mittel sind, die geeignet sind, während einer Dauer, welche wenigstens gleich der Dauer der Phase der Vermutung einer Störung des Herzvorhof-Rhythmus des Patienten ist, die Überprüfung der Bedingungen a) bis c) der Kriterien zur Umschaltung vom AAI-Modus zum DDD-Modus auszusetzen, bei Beibehaltung der Überprüfung der Bedingung d).

2. Vorrichtung nach Anspruch 1, bei welcher die Mittel zur Zustandserfassung Mittel sind, die geeignet sind, außerdem eine Phase einer Herzdekompensation des Patienten zu diagnostizieren.

3. Vorrichtung nach Anspruch 1, bei welcher die Mittel zur Modusumschaltung eine Batterie an Zählern umfassen, die jeweils den Bedingungen a) bis d) der Kriterien zur Umschaltung vom AAI-Modus zum DDD-Modus und den Zustandstypen, die diagnostiziert werden können, zugewiesen sind, wobei der entsprechende Zähler inkrementiert wird, wenn die jeweilige Bedingung bestätigt ist und der jeweilige Zustandstyp erfasst ist.

4. Vorrichtung nach Anspruch 3, bei welcher die Mittel zur Modusumschaltung eine Batterie an Schwellen umfassen, die der Batterie an Zählern entspricht, wobei jede Schwelle programmierbar ist, und geeignet sind, das Umschalten vom AAI-Modus zum DDD-Modus zu erzwingen, wenn wenigstens eine der Schwellen durch den entsprechenden Zähler erreicht wird.

5. Vorrichtung nach Anspruch 1, bei welcher die Mittel zur Modusumschaltung Mittel sind, die in Antwort auf die Mittel zur Zustandserfassung arbeiten, um die Kriterien der Rückkehr zum AAI-Modus nach Umschaltung in den DDD-Modus während der Zeit zu verändern, wo eine Anstrengungs- oder Schlafphase diagnostiziert wird.

6. Vorrichtung nach Anspruch 5, bei welcher die Mittel zur Modusumschaltung Mittel sind, die in Antwort auf die Mittel zur Zustandserfassung arbeiten, um vorübergehend jegliche Rückkehr zum AAI-Modus nach Umschaltung in den DDD-Modus während der Zeit auszusetzen, wo eine Anstrengungs- oder Schlafphase diagnostiziert wird.

7. Vorrichtung nach Anspruch 5, bei welcher die Mittel zur Modusumschaltung Mittel sind, die in Antwort auf die Mittel zur Zustandserfassung arbeiten, um vorübergehend die Kriterien der Rückkehr zum AAI-Modus nach Umschaltung in den DDD-Modus in eine restriktive Richtung während der Zeit zu verändern, wo die Anstrengungs- oder Schlafphase diagnostiziert wird.

8. Vorrichtung nach Anspruch 7, bei welcher die Mittel zur Modusumschaltung Mittel sind, die in Antwort auf die Mittel zur Zustandserfassung arbeiten, um ohne Verzögerung die Rückkehr zum AAI-Modus nach Umschaltung in den DDD-Modus nach dem Ende der Anstrengungs- oder Schlafphase anzuordnen.

9. Vorrichtung nach Anspruch 7, bei welcher die Mittel zur Modusumschaltung Mittel sind, die in Antwort auf die Mittel zur Zustandserfassung arbeiten, um vorübergehend die Kriterien der Rückkehr zum AAI-Modus nach Umschaltung in den DDD-Modus nach dem Ende der Anstrengungs- oder Schlafphase in eine extensive Richtung zu verändern.

10. Vorrichtung nach Anspruch 1, bei welcher:

- die vorbestimmten Kriterien ebenfalls folgendes umfassen:

■ Kriterien zur dauerhaften Umschaltung in den DDD-Modus, und

- die Mittel zur Modusumschaltung ebenfalls Mittel zur dauerhaften Umschaltung umfassen, die geeignet sind, die Rückkehr vom DDD-Modus zum AAI-Modus zu unterdrücken, und den Betriebsmodus in Abhängigkeit der Kriterien zur dauerhaften Umschaltung zum DDD-Modus zu zwingen.

**11.** Vorrichtung nach Anspruch 10, bei welcher die Mittel zur dauerhaften Umschaltung Zähler umfassen, die selektiv in Abhängigkeit der Bedingungen a) bis d) der Kriterien zur Umschaltung vom AAI-Modus zum DDD-Modus inkrementiert werden, wenn die jeweilige Bedingung bestätigt ist, und Mittel zur Unterdrückung der Rückkehr vom DDD-Modus zum AAI-Modus und zur Zwingung des Betriebsmodus zum DDD-Modus, wenn die Zähler eine vorbestimmte Zählkonfiguration erreichen.

**12.** Vorrichtung nach Anspruch 11, bei welcher die Mittel zur dauerhaften Umschaltung geeignet sind, die Inkrementierung der Zähler zu unterdrücken, wenn eine der Bedingungen a) bis c) bestätigt ist, bei Gestattung der Inkrementierung, wenn die Bedingung d) bestätigt ist, im Falle einer Umschaltung, die sich an eine Diagnose einer Anstrengungs- oder Schlafphase anschließt.

**13.** Vorrichtung nach Anspruch 11, bei welcher die Mittel zur dauerhaften Umschaltung geeignet sind, die Inkrementierung der Zähler im Falle des Erfolgens einer Umschaltung zu unterdrücken, die von der vorangehenden Umschaltung durch eine Anzahl an Zyklen im AAI-Modus getrennt ist, die geringer als eine fünfte vorbestimmte Zahl ist.

**14.** Vorrichtung nach Anspruch 10, bei welcher die Mittel zur dauerhaften Umschaltung geeignet sind, die Rückkehr vom DDD-Modus zum AAI-Modus zu unterdrücken, und den Betriebsmodus zum DDD-Modus zu zwingen, wenn die Anzahl an Umschaltungen vom AAI-Modus zum DDD-Modus über eine erste vorbestimmte Zeitperiode größer als eine sechste vorbestimmte Zahl ist.

**15.** Vorrichtung nach Anspruch 10, bei welcher die Mittel zur dauerhaften Umschaltung geeignet sind, die Rückkehr vom DDD-Modus zum AAI-Modus zu unterdrücken, und den Betriebsmodus zum DDD-Modus zu zwingen, wenn der Prozentsatz an stimulierten Herzkammerereignissen über eine zweite vorbestimmte Zeitperiode höher als eine erste vorbestimmte Schwelle ist.

**16.** Vorrichtung nach Anspruch 10, bei welcher die Mittel zur dauerhaften Umschaltung Mittel sind, die geeignet sind, am Ende einer jeden Schlafphase die Rückkehr zum Betriebsmodus im AAI-Modus anzuordnen.

**17.** Vorrichtung nach Anspruch 11, bei welcher die Mittel zur dauerhaften Umschaltung Mittel sind, die geeignet sind, am Ende einer jeden Schlafphase die Zähler zu reinitialisieren.

**18.** Vorrichtung nach Anspruch 10, bei welcher:

- die vorbestimmten Kriterien ebenfalls folgendes umfassen:

■ Kriterien zur definitiven Umschaltung in den DDD-Modus, und

- die Mittel zur Modusumschaltung ebenfalls Mittel zur definitiven Umschaltung umfassen, die geeignet sind, die Rückkehr vom DDD-Modus zum AAI-Modus zu verbieten und bis zur Reprogrammierung der Vorrichtung einen DDD-Betriebsmodus vorzuschreiben, in Abhängigkeit der Kriterien zur definitiven Umschaltung.

**19.** Vorrichtung nach Anspruch 18, bei welcher die Mittel zur definitiven Umschaltung geeignet sind, die Rückkehr vom DDD-Modus zum AAI-Modus zu verbieten und bis zur Reprogrammierung der Vorrichtung einen DDD-Betriebsmodus vorzuschreiben, wenn die Anzahl an dauerhaften Umschaltungen vom AAI-Modus zum DDD-Modus über eine dritte vorbestimmte Zeitperiode größer als eine siebte vorbestimmte Zahl ist.

**20.** Vorrichtung nach Anspruch 18, bei welcher die Mittel zur definitiven Umschaltung geeignet sind, die Rückkehr vom DDD-Modus zum AAI-Modus zu verbieten und bis zur Reprogrammierung der Vorrichtung einen DDD-Betriebsmodus vorzuschreiben, wenn über eine vierte vorbestimmte Zeitperiode der Prozentsatz an stimulierten Herzkammerereignissen größer als eine zweite vorbestimmte Schwelle ist.

**21.** Vorrichtung nach Anspruch 1, bei welcher die Mittel zur Modusumschaltung die erste Bezugsdauer während der Zeit, wo eine Anstrengungsphase diagnostiziert wird, verringern.

**22.** Vorrichtung nach Anspruch 21, bei welcher die Mittel zur Modusumschaltung die erste Bezugsdauer auf einen Wert verringern, der eine Kehrwertfunktion der Stimulationsfrequenz ist.

**23.** Vorrichtung nach Anspruch 22, bei welcher die Kehrwertfunktion von der Grundstimulationsfrequenz bis zur maximalen Stimulationsfrequenz eine monoton abnehmende Funktion ist.

24. Vorrichtung nach Anspruch 22, bei welcher die Kehrwertfunktion von einer niedrigen Frequenz, die höher als die Grundstimulationsfrequenz ist, bis zu einer hohen Frequenz, die höher als die maximale Stimulationsfrequenz ist, eine monoton abnehmende Funktion ist, und unterhalb der Grundstimulationsfrequenz und oberhalb der maximalen Stimulationsfrequenz eine konstante Funktion ist.

25. Vorrichtung nach Anspruch 1, weiterhin mit:

- Mitteln zur differenzierten Speicherung von Herzdaten, die geeignet sind, während der Zeit, wo der vorübergehende spezifische Zustand diagnostiziert wird, spezifisch gewisse vorbestimmte Daten zu speichern.

26. Vorrichtung nach Anspruch 25, bei welcher die durch die Mittel zur differenzierten Speicherung gespeicherten vorbestimmten Daten die Werte der Zeitspannen der atrio-ventrikulären Leitung umfassen.

27. Vorrichtung nach Anspruch 25, bei welcher die durch die Mittel zur differenzierten Speicherung gespeicherten vorbestimmten Daten die Dauer von Episoden umfassen, während welcher Umschaltungen aufeinander folgen, die alle voneinander um eine Anzahl an Zyklen im AAI-Modus getrennt sind, die geringer als eine fünfte vorbestimmte Zahl ist.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0488904 A
- EP 1346750 A
- US 20020082646 A
- US 20030078627 A
- US 2003078627 A
- EP 0755696 A
- EP 0838235 A
- EP 0550293 A
- EP 0750920 A
- EP 0719568 A
- EP 0770407 A